# EUROPEAN PATENT APPLICATION

(11) **EP 0 979 828 A1**
(43) Date of publication of application: **16.02.2000**
(21) Application number: 99114963.4
(22) Date of filing: 30.07.1999
(51) Int. Cl.: C07K 5/097, C07K 5/103, C07K 5/117, G01N 33/68

(54) **A method for the diagnosis of autism**

(30) Priority: 04.08.1998 IT MI981834
(71) Applicant: Radim S.p.A., 00040 Pomezia (Roma) (IT)
(72) Inventor: Reichelt, Karl L., 00040 Pomezia (Roma) (IT); Pedersen, Odd S., 00040 Pomezia (Roma) (IT); Persico, Antonio Maria, 00040 Pomezia (Roma) (IT); Keller, Flavio, 00040 Pomezia (Roma) (IT); Ying, Liu, 00040 Pomezia (Roma) (IT)
(74) Representative: Minoja, Fabrizio, Dr.

(57) **Abstract**

A method for the diagnosis of autism, which comprises detecting a peptide of the invention in a biological fluid.

## Description

### DISCLOSURE OF THE INVENTION

The present invention relates to a method for the diagnosis of psychiatric disorders which can be related to autism.

More particularly, the invention relates to a method for the diagnosis of autism and related disorders based on the determination of novel peptides, which are a further object of the invention, in a biological fluid of the patients.

Infantile autism is a psychiatric disorders heterogeneous from the point of view of the pathogenetic mechanisms. Such pathology, also known as Kanner's syndrome, usually arises in early childhood, within the first two or three years of life, and is characterized by language disorders, incapability of developing social relationships, rituals and repetitive behaviours and, often, mental retardation.

The diagnosis of autism is usually carried out by clinical observation of the child, as today no biochemical parameters which can be considered reliable markers from the prognostic/diagnostic point of view are available.

Diagnosis is complicated by the fact that other disorders of the nervous system can have symptoms very similar to autism, and diagnosis can be made only after repeated, prolonged observations of the patient by experienced examiners.

In spite of the strong genetic components of this disease, no genetic or biochemical alterations responsible for this pathology have so far been detected.

US 5,686,311 discloses a method for diagnosing autism based on the determination of a series of metabolites, such as citramalic acid, tartaric acid, furan-2,5-dicarboxylic acid, 3-oxo-glutaric acid, arabinose, dihydroxyphenylpropionic acid, furancarbonylglycine, carboxycitric acid, 5-hydroxymethyl-2-furoic acid.

The high levels of said metabolites in autistic patients are hypothetically explained as a possible result from a fungal infection, in that the administration of antifungal drugs to autistic children was found to normalize metabolites levels as well as to improve symptomatology.

Whether this hypothesis is correct or not, it is anyway unquestionable that the determination of such large number of metabolites, every one of them being per se insufficient to discriminate a physiological condition from a pathological one, does not yet provide a satisfactory solution to the problem of diagnosing autism by means of biochemical markers.

Reichelt et al. (*Biol. Psychiatry,* 1986, 21 (13) 1279-90; *Adv. Biochem. Psychophazmacol.,* 1981, 28, p. 627-43; *Med. Hypotheses,* 1995, 45 (5), p. 498-502) detected peptiduria in autistic children and purified some of the peptides present in urines of autistic patients, in an effort to identify patients in which the autistic syndrome is caused by a common pathogenetic mechanism.

Peptides have now been identified which are present at higher frequency in autistic patients and therefore can be considered sensitive, reliable markers for the diagnosis of autism.

The peptides of the present invention have the following sequences:

pyroGlu-Trp-GlyNH2  Gly-Ser-Glu-Asn  pyroGlu-Glu-Asp-Ser
E*-W-G°  G-S-E-N  E*-E-D-S
* pyroglutamic acid
o amidated glycine

Said peptides, purified to homogeneity by biochemical procedures, acutely stimulate serotonin (5-HT) uptake in vitro in platelets from human blood in picomolar concentrations.

The determination of the peptides of the invention for diagnostic purposes can be carried out in any biological fluid, but preferably in urine or blood.

Quantitative determination of the peptides of the invention in urine can conveniently be effected, besides by chromatographic techniques (HPLC), also by immunoassays, which make use of an antibody specifically against the peptide. Such procedures are well known to those skilled in the art and have successfully been applied to the analysis of biological samples (serum, urine, etc.) for dosing a large number of analytes of protein, peptide, steroid natures or of drugs. A thorough review on the state of the art concerning such procedures can be found in the book by P. Tijssen "Practice and Theory of Enzyme Immunoassays", Elsevier, Amsterdam 1985.

The main advantages of immunoassays are easiness of operation, short operational time and, above all, possibility of carrying out a large number of analysis with reduced use of staff and instrumentation. A further advantage of immunoassays compared with chromatographic techniques is their high specificity and sensitivity, which allows for biological samples to be directly analysed without undergoing procedures for extracting the analyte.

In order to obtain antibodies against low molecular substances, as in this case, the selected animal should be immunized with a chemically stable conjugate formed by the compounds of interest, and high molecular weight carrier, which gives the conjugate the required immunogenicity. Bovine serum albumin (BSA), thyreoglobulin, ovoalbumin or synthetic proteins (polylysin).

The procedures for the preparation of said conjugates have been known for some time and described in scientific literature (Glazer A.N., et al.: Selected methods and Analytical Procedures, North-Holland, Amsterdam, 1975; Erlanger B.F., Methods Enzymol., 70: 85, 1980; Tateishi K. et al., Steroids, 30: 25, 1977; Carlsson J.H. et al., Biochem. J., 173: 723, 1978).

The conjugates obtained according to the above mentioned procedures are used to prepare either polyclonal (usually from rabbit) or monoclonal (usually from mouse) antibodies: both types can be used to develop immunoassays.

Among the various immunoassay methods described, the most widely used in the case of low molecular weight compounds such as the peptides of the invention is the competitive method, in which the peptide is immobilized on a solid phase and the one present in the sample competes for binding with a given amount of antibody. For detecting binding, the antibody, previously purified by conventional techniques (Steinbuch M. et al., *Arch. Biochem. Biophys.,* 134 : 279, 1969; McKinney M.M. et al., *J. Immunol. Methods,* 96 : 271, 1987) should be either radiolabeled (e.g. with ¹²⁵I), or labeled with an enzyme (e.g. horseradish peroxidase or alkaline phosphatase) or with a fluorescent or chemiluminescent molecule, according to procedures described in literature (Greenwood F. et al., *Biochem J.,* 89 : 114, 1963; Nakane P.K. et al., *J. Histochem. Cytochem.,* 22 : 1084, 1974; Kronick M.N., *J. Immunol. Methods,* 92 : 1, 1986).

The solid phase for use in an immunoassay can be any insoluble material capable of fixing at its surface the peptide conjugated with a carrier and of being contacted with the sample to be analysed and with the specific labeled antibody as mentioned above. In the most widely used forms, this solid phase is a plastic material (polystyrene, polypropylene) in the form of beads, test-tubes, microplates or microbeads in suspension (latex). Also modified plastics can be used, having at their surface reactive groups (carboxylic, aldehyde or amino) which can form a covalent bond between the solid phase and the peptide of the invention, even without the intermediate carrier.

The analytical sensitivity of the immunoassays can be varied within wide ranges, so as to make it suitable to the specific requirements, by suitably varying the concentrations of the reactants. In order to quantify the analyte concentration in the sample, a calibration curve can be inserted in the assay, consisting of known, scalar concentrations of the analyte itself, which allows to determine by comparison the concentration of the analyte in the tested sample.

The following examples further illustrate the invention.

### EXAMPLE 1

### Procedure for isolating from blood and urine the oligopeptides stimulating serotonin platelet uptake in vitro.

Starting material: 80 ml of blood or 2 1 of urine.
a) First separation of the peptides from amino acids and salts by means of a C-18 preparative column (this step being carried out for urine only);
b) Separation of macromolecules from low molecular weight molecules by gel filtration in G-25 columns;
c) Isolation of the fraction with activity stimulating serotonin platelet uptake in vitro;
d) Separation of the peptides from salts, amino acids, ammonia and urea by C-18 reverse phase on preparative column;
e) Freeze-drying and elution of the material present in the active fractions on a Dowex 50 ion exchange column;
f) Low molecular gel filtration on Fractogel MG2000 of the non-protonable, active fraction from step e);
g) Anion exchange, by DEAE columns with cellulose filter which do not retain the biological activity;
h) Cation exchange, by DOW 50 columns;
i) Analytical HPLC carried out on a C-18 reverse phase column by 4 different gradients;
j) Hydrolysis in 6M HCl and analysis of the amino acid sequence by an amino acid analyzer; mass spectrometry.

### EXAMPLE 2

### Stimulation of serotonin uptake.

The capability of the peptide pyroGlu-Trp-GlyNH₂ of stimulating 5-HT reuptake was measured in an in vitro system consisting of CHO.K1 cells transfected with the corresponding cDNA at the serotonin human carrier.

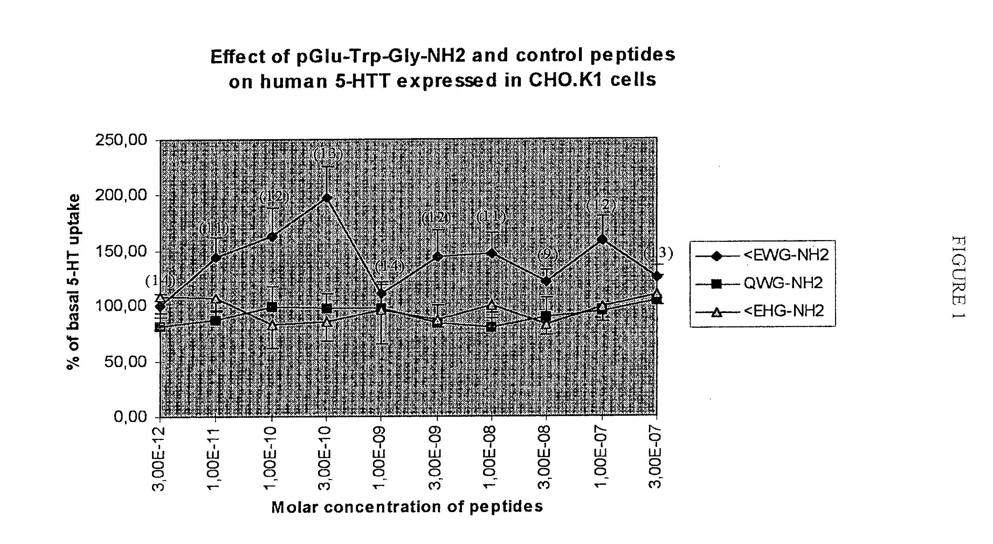

Peptides in which the first or second amino acid had been mutated completely lost the capability of stimulating 5-HT reuptake. Results are reported in Figure 1.

### EXAMPLE 3

### Frequency and quantity of pyroGlu-Trp-GlyRH₂ in autistic patients.

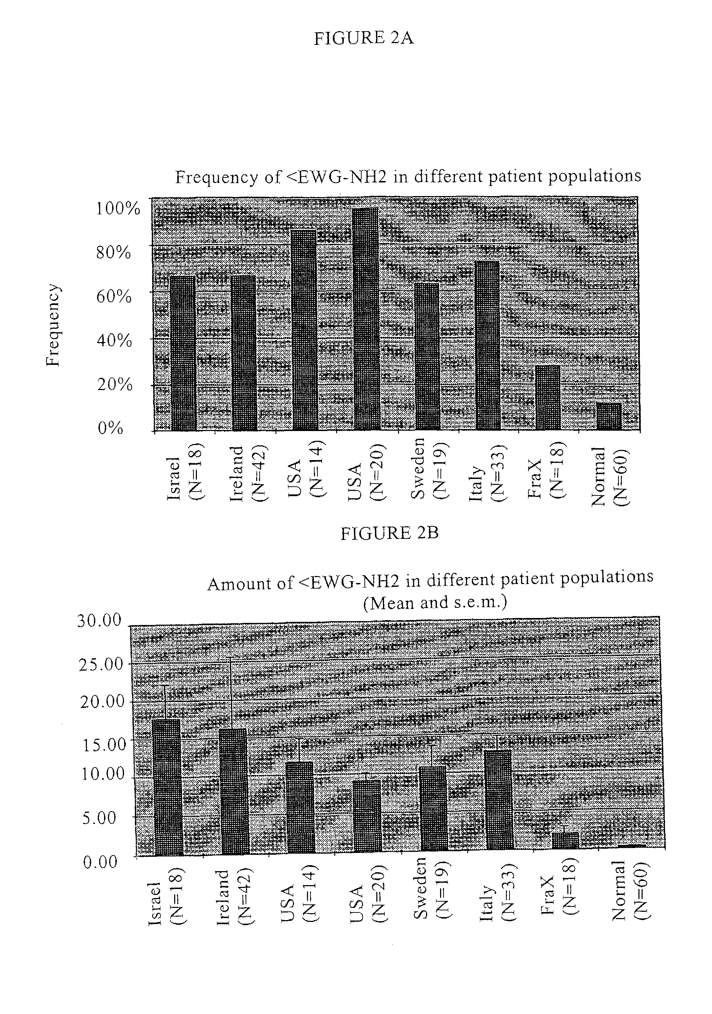

An HPLC analysis of urine from autistic patients allowed to identify an absorbance peak having the same characteristics (retention time) as the synthetic peptide pyroGlu-Trp-GlyNH₂. This peak has been quantified by integrating the area under the peak. An analysis performed on urine from groups of patients from different Countries showed that the frequency of the cases in which the peptide can be detected in urine, as well as the amount of the peptide, remarkably increase in autistic patients compared with normal controls and patients with fragile X syndrome (FraX), a psychiatric disorder with clinical phenotype partially similar to autism, but of different origin (Figures 2a and 2b).

## Claims

1. Peptides of formulae:
pyroGlu-Trp-GlyNH₂, Gly-Ser-Glu-Asn and pyroGlu-Glu-Asp-Ser.

2. Peptides as claimed in claim 1 as diagnostic markers for autism.

3. A method for the diagnosis of autism, which comprises the determination of one of the peptides of claim 1 in a biological fluid of a patient.

4. A method as claimed in claim 3, wherein the biological fluid is blood or urine.

5. A method as claimed in claim 3 or 4, wherein the determination is carried out by a chromatographic method.

6. A method as claimed in claim 3 or 4, wherein the determination is carried out by an immunological method.
